# EUROPEAN PATENT SPECIFICATION

(11) **EP 0 292 995 B1**
(45) Date of publication and mention of the grant of the patent: **18.11.1993**
(21) Application number: 88108515.3
(22) Date of filing: 27.05.1988
(51) Int. Cl.: C12M 1/32, B01L 3/02

(54) **Cell passage device**
Vorrichtung zur Übertragung von Zellen
Appareil pour le transfert de cellules

(30) Priority: 29.05.1987 JP 136729/87
(43) Date of publication of application: 30.11.1988
(73) Proprietor: SUMITOMO ELECTRIC INDUSTRIES LIMITED, Osaka-shi, Osaka 541 (JP)
(72) Inventor: Miyake, Shinichi, c/o Osaka Works, Konohana-ku, Osaka-shi, Osaka-fu (JP)
(74) Representative: Ritter und Edler von Fischern, Bernhard,Dipl.-Ing.

(56) References cited:
- EP-A- 0 195 088
- FR-A- 2 530 814
- US-A- 3 143 393
- US-A- 4 198 483
- PATENT ABSTRACTS OF JAPAN, vol. 11, no. 332 (C-455)[2779], 29 October 1987#

## Description

### (a) Field of the Invention

The present invention relates to a cell passage device for grafting cells in a cell culturing process.

### (b) Description of the Prior Art

It is often observed that cultured cells die when they continue to grow in a culture vessel such as a microplate or dish and reach to an excessive concentration called "full growth". Accordingly, the cells which have almost grown to the full growth are partly transferred to a fresh culture vessel, where the transferred cells are subcultured after being diluted with a culture medium.

For the cloning of the cells, a limiting dilution method is also employable. In this method, cultured cells in a vessel are transferred to a microplate so that not more than one cell may be added to each well of the plate, for instance in the ratio of one cell/two wells, and the subculture is continued. More particularly, the cell concentration in the culture vessel is determined by analyzing a cell sample drawn from the vessel using a hemocytometer. On the basis of the determined cell concentration, the cell culture is diluted such that the concentration may be reduced to 2.5 cells /ml where one cell/two wells is desired, or 1.7 cells/ml where one cell/three wells is desired, and the diluted culture is spread on a microplate.

It is known to provide movable pipette to fill vessels with liquid solutions, or to extract such liquid solutions. This is shown in FR-2 530 814, EP-0 195 088 and US-3 143 939.

### SUMMARY OF THE INVENTION

An essential object of the present invention is to provide a cell passage device which is able to transfer the cells without full growth, whereby death of the cells can be effectively prevented.

Another object of the present invention is to provide a cell passage device which is operable automatically to transfer the cells preventing the problem of the full growth of the cell.

A further object of the present invention is to provide a cell passage device which is operable with a simple operation to transfer the cells with a high efficiency.

According to the present invention there is provided a cell passage device comprising: a pipette manipulator having its end mounted with a pipette and movable in three dimensions; means for sucking and discharging solution to a culture solution vessel; a first cell solution vessel for retaining cell solution; a second cell solution vessel for receiving the cell solution of the first vessel through said pipet manipulator, characterized by means for controlling the pipette and the sucking and discharging means, so as to agitate cell solution in the first or second cell solution vessel.

### BRIEF DESCRIPTION OF THE DRAWINGS

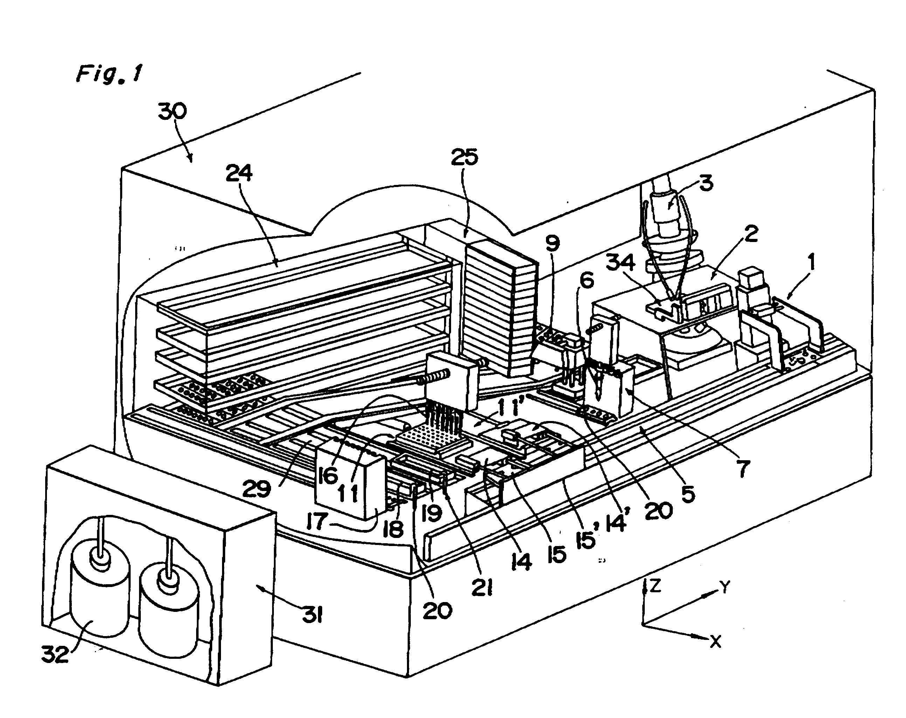

Fig. 1 is a perspective view showing an example of a cell passage device according to the present invention.

### DETAILED DESCRIPTION OF THE INVENTION

A tray transferring conveyer 5 comprises a conveyer (not shown), a tray centering mechanism and a tray stopper. The tray centering mechanism acts to move the tray in a direction perpendicular to the movement of the conveyer to position the tray in position on the conveyer. The conveyer stopper acts to position a tray of a generally rectangle shape in a direction parallel to the movement of the conveyer by contacting the tray to a stopper member so as to prevent overrunning of the tray. The operation of the tray centering mechanism and tray stopper enables to transfer the tray to various mechanism provided in the cell passage device at every correct position. A two phase motor is used for driving the conveyer and the rotation of the motor is transmitted through a pulley and deceleration mechanism to another pulley which drives a conveyer belt. The tray centering mechanism has a rotary air cylinder as a driving source and the rotation of the rotary air cylinder is converted to a linear motion by a crank mechanism so that said pair of the clamp members clamp the tray from both sides to set the tray at a centering position. As the tray stopper, a linear air cylinder is used.

The cell culturing tray having 96 wells for receiving the respective cell containers is transferred to a lid mounting and removing mechanism 15.

In the cell passage device according to the present invention, there are provided two tray work-stations 11 and 11′juxtaposed each other and two sets of the lid mounting and removing mechanism 15 and 15′ each having the same structure are provided in positions corresponding to the respective tray work-stations 11 and 11′. Each of the lid mounting and removing mechanisms 15 and 15′ comprises a clamp mechanism, one or more vacuum pads and an elevator for moving up and down the vacuum pads. The clamp mechanism has a pair of clamp members each driven by an air cylinder so that said pair of the clamp members clamp the across corners of the tray for positioning the tray at a desired position for removing and mounting the lid.

The 96 well culturing tray having its lid removed by the lid mounting and removing mechanism 15 (or 15′) is moved to the working area 11 by the tray transferring mechanism 14. As shown in the Figure 1, there are provided a pair of tray transferring mechanisms 14 and 14′ having the same structure corresponding to the respective tray rid removing mechanisms 15 and 15′.

Each of the tray transferring mechanisms 14 and 14′ are composed of a clamp mechanism for clamping the tray situated on the transmission conveyer 5, a lifter for lifting the tray clamped by the clamping mechanism on the workbench, a carrier for carrying the tray on the workbench and a positioning mechanism for positioning the tray in a longitudinal direction of the tray.

The clamp mechanism comprises clamp members for clamping the tray at the opposite ends in the longitudinal direction of the tray. The positioning mechanism is provided with one or more free rollers and spring members to push the free rollers so as to position the tray smooth in the longitudinal direction. The position of the tray in the lateral direction can be decided by a stopper. The lifter and carrier are respectively constructed by linear air cylinders.

A 24 well tray is also transferred to any one of the work-stations 11 and 11' by the conveyer 5 with the lid removed.

A predetermined amount of culture solution stored in a bottle 32 situated in a refrigerator 31 with a temperature of 4°C is fed to a solution tank 18 through a pipette 20.

A pipette 6 of 1 ml is moved to a well in which cells are propagating and repeats suction and discharge of the cell culture solution several times so as to stir the cell culture solution in the well. If the amount of suction of the pipet eyceeds the amount of discharge of the pipette 6, there occur air bubbles in the solution, therefore the amount of discharge should be smaller than the amount of the suction. In the example, the amount of suction was 150 µℓ and the amount of discharge was 150 µℓ. The number of time of stir was 5.

The pipette 6 has its end attached to a 1000 µℓ tip. The tip may be changeable. In order to take the solution of the various wells, the pipet 6 is mounted on a movable base which is moved in X direction by an X direction driving unit and in Y direction by a Y direction driving unit.

In order to move the tip in a vertical or Z direction, there is provided a Z direction driving unit. In order to suck and discharge the solution, the cylinder of the pipet 6 is moved by a cyridine pump driven by a pulse motor. There is further provided a locking mechanism for locking the vertical motion of the cylinder of the pipet 6.

The X direction drive unit is composed of a slide block movably mounted on a ball screw extending in the X direction and a linear slide shaft and the ball screw is driven by a pulse motor.

The pipette 6 which is single pipet is provided with a tip adapter which is made of a cubic block having six through holes for accommodating the respective tips. When all of the tips in the adapter have been used, the adapter is exchanged by a new adapter so that new tips can be used for the pipet 6.

A tip exchanger 7 is composed of a tip adapter moving unit, a tip adapter transferring unit, tip adapter discharging unit and a tip adapter mounting unit.

A new tip or tips are accommodated in a tip stocker 9 under such a condition that the tips are inserted in the tip adapter and the tip adapter moving unit takes out the tip adapter from the tip stocker 9. The tip adapter now taken out is carried to the tip adapter transferring unit. The tip moving unit is composed of a clamping unit for clamping the tip adapter, a rotating unit for rotating the clamping unit, a shifting unit for shifting the clamping unit in forward and reverse directions or a direction parallel to the conveyer 5 (Y direction), an elevating unit for moving the clamping unit, rotating unit, shifting unit and elevating unit in the vertical direction and a displacing unit for displacing the respective units to the tip adapter transferring unit.

The clamping unit is adapted to clamp the tip adapter at both longitudinal ends of the adapter by a pair of clamping members which are moved by an air cylinder. The shifting unit and displacing unit are respectively moved by air cylinders. The rotating unit is rotated by a rotary air cylinder through a deceleration mechanism.

The tip adapter transferring unit transfers the tip adapter between the tip mounting position and tip adapter moving unit. In this unit, the tip adapter is carried by a belt conveyer which is driven by a DC motor.

The tip adapter discharging unit is situated below the tip stocker 9 and acts to discharge unnecessary tips to the outside of the device.

The tip adapter mounting unit acts to mount the tip transferred by the tip adapter transferring unit to the pipet 6 and to dismount the tip therefrom. The tip adapter mounting unit comprises an elevation unit for moving the tip adapter up and down, a positioning unit for mounting the tip adapter on the tip adapter mounting unit and a dismounting unit for dismounting unnecessary tips from the pipette 6. The tip adapter has four guide holes made of through holes at the four corners of the adapter. Four poles provided in the tip adapter mounting unit are inserted in the four guide holes to maintain the relative position between the pipet 6 and the tip. The positioning unit acts to position the poles at a position where the poles can be correctly inserted in the four guide holes of the tip adapter.

The elevation unit comprises a slide shaft and a ball screw standing in the vertical direction the ball screw being driven by a pulse motor so that the rotating of the ball screw causes an elevating member to be moved up and down. When the unnecessary tips are removed from the pipet 6, a tip removing plate of a comb shape is inserted in the upper portion of the tip to clamp the tip by the removing plate and the tip clamped by the removing plate falls down by an air cylinder, whereby the tip is removed from the pipet and restored in the tip adapter. The tip exchanging mechanism mentioned above is used when different cells in a separate well are passaged.

The culture solution contained in the well of the 96 well culture tray is agitated and after the agitation is stopped the culture solution in the well is left for a suitable time so that only the living cells are deposited to the bottom of the well using the difference of density between the died cells and living cells and subsequently the supernatant is thrown away, whereby the concentration of the living cells in the culture solution is increased. In the present example, the agitated well was left for five minutes, subsequently 150 µℓ of the supernatant was thrown away in the tank 19 by the pipette 6. Subsequently 150 µℓ of the cultule solution stored in the tank 18 was added using the pipette 6 to the well from which the supernatant was removed. This process was made to increase the degree of dilution of the culture solution because the concentration of the cell after cell growth is usually 10⁵ to 10⁶ cells/mℓ.

Subsequently, the solution in the well is agitated by the pipette 6 under the same condition mentioned above. After the agitation, a part of the cell culture solution is taken by the pipette 6 and the same amount of the cell culture solution is delivered to two wells of the 24 well tray. In the present example, 100 µℓ of the cell culture solution was taken out of the agitated well and 50 µℓ of the cell culture solution is delivered to one well for counting the number of cells and to another well for delivering the cell culture solution in the limiting dilution.

Subsequently the cell culture solution is added in the two wells and the solution in the two wells are again agitated. The agitation mentioned above is made by repeating sucking and discharging of the solution by the pipette 6. Since the area of the well of the 24 well tray is large, the solution can not be sufficiently agitated so far as the suction and discharge by the pipette 6 are repeated in the same position, and so the pipette 6 is moved as mentioned above. In the present example, 350 µℓ of the culture medium was added to the well for counting the number of the cells and suction and discharge were repeated at the center of the well and four positions separated by 90° on the respective positions between the center of the well and the peripheral wall of the well, whereby the agitation is made at the five positions. At every position, the amount of suction was 200 µℓ, the amount of discharge was 200 µℓ and the suction and discharge were repeated five times. 950 µℓ cell culture solution was added to the limiting dilution well.In this step, a lid is put on the 96 well tray, which is transferred to the transfer conveyer 5 and removed from the passage device. A suitable amount of Nigrosine dyeing solution may be added to the well of the measurement of the 24 well tray as desired.

The 24 well tray of which the measurement well is already agitated is covered with a lid by the lid mounting mechanism 15 in a reversed procedure performed when the tray is moved to the working area 11. Then the 24 well tray is put on the conveyer 5 and is transferred to a tray moving mechanism 1. The tray moving mechanism 1 receives the tray from the conveyer 5 and carries the tray to a microscope stage 2 . Also the tray moving mechanism 1 transfers the tray to the conveyer 5 from the microscope 2. The tray moving mechanism 1 comprises a clamp unit for handling the tray and a vertical shaft moving mechanism and rotation shaft moving mechanism for lifting and carrying the tray from the microscope to the conveyer. The clamp unit is such an arrangement that a finger is directly driven by the output shaft of an air cylinder and another finger is driven by the output shaft of the air cylinder through a gear so that both fingers clamp the tray. The vertical shaft moving mechanism is composed of a ball screw and a linear slide shaft provided in parallel with the ball screw driven by a 2 phase pulse motor. The rotation shaft moving mechanism has its rotation axis situated on the vertical shaft and disposed on a base for supporting the vertical shaft so as to rotate the clamp unit and the vertical shaft. The rotation shaft is driven by a motor situated on a clean bench base through a deceleration mechanism formed by a belt and a pulley.

The 24 well tray carried to the microscope stage 2 is rest for a period until the cells in the well which has been agitated stop their motion. In the present example, the tray was rest for 10 minutes. After the cells are stabilized, the tray is transferred to the measurement position of the microscope 3 by a scanning mechanism 34.

The scanning mechanism 34 acts to move the tray between the tray receiving position of the tray moving mechanism 1 and the measuring position of the microscope 3 and comprises a clamp unit for clamping and handling the tray and a X shifter and Y shifter for moving the tray in a rectangular coordinate. The clamp unit is the same structure as that of the tray moving mechanism. The Y shifter moves the clamp unit and comprises a ball screw and a slide rail. The X shifter supports the Y shifter in a canti lever manner for moving the Y shifter in the arrangement using a ball screw and a slide rail. In order to operate the shifters in a high precision, a 5 phase pulse motor is used.

The microscope 3 is provided with a television camera and the picture information of the microscope 3 obtained in the television camera is processed by a picture processing device so as to count the number of the living cells. The way of counting the number of the living cells is disclosed in the Japanese patent application 61-124411 entitled "method of monitoring the cell culture". When the focus position of the well is not known, it is desired to add an automatic focusing apparatus using the phase difference system and contrast system. In the present example, the number of the cells was counted using the automatic focus control system by taking 49 pictures of 1.1 mm x 1.2 mm wide picture element in the well and scanning of the picture elements was made by the scanning mechanism 34.

The 24 well tray in which the number of the cells have been counted is returned to the working area 11'. Subsequently another 96 well tray is put on the conveyer 5 and transferred to the work-station 11 through the lid mounting and removing unit 15 and tray moving mechanism 14. The solution tanks 18 and 19 are replaced by new tanks by the tank transfer mechanism 29.

The tank transfer mechanism 29 is provided to transfer the tank to the solution supplier. The cell solution and culture solution saved in the refrigerator 31 are once stored in the tank then poured to the tray by the pipet 6 and 16. The solution tank is used to receive the discharged solution from the well. The tank transfer mechanism 29 comprises a tank conveyer, a tank exchanger, a tank stopper and a solution meter. The tank is mounted on the tank conveyer from the tank stocker 25 and moved to the tank exchanger. There are provided two tank positions 18 and 19. In order to carry the tank in the tank position 18 (or 19), the tank is moved by the tank conveyer until the tank is stopped by a mechanical stopper. When the unnecessary tank is discharged, the tank is put on the tank conveyer and carried to the tank exchanger and further transferred to the outside of the cell passage device by the tank conveyer. The tank conveyer is driven by a DC motor. The tank exchanger is composed of an exchange table which is rotated around one edge of the table as a rotation axis by an air cylinder so that the table is moved up and down to charge and discharge the tank. The tank exchanger may be moved up and down through a crank mechanism driven by an air cylinder. The tank stopper also may be moved by an air cylinder.

The solution meter is provided with a measuring table for weighing the amount of the solution and when the solution in the tank is measured, the measuring table with the tank is lifted from the conveyer so that the tank is loaded on a load cell and the amount of the solution is measured by the output information of the load cell. In order to move the measuring table, an air cylinder is used. After the solution is measured, the new tank is replaced by the old tank.

Dilution to the tray is performed according to a dilution rate calculated by the measurement value of the measurement well of the 24 well tray. In this example, the number of the cell to be added was 0.5/well, the number of the cell in the original well was 10⁶/mℓ, therefore the dilution was performed in the manner mentioned below.

The cell solution in the 24 well tray for the limiting dilution was agitated in the manner mentioned in the process of agitation of the well for the cell counting. The agitated cell solution of 780 µℓ poured in the tank 19 by the pippet 6. In addition, 24220 µℓ of the culture solution was added to the tank 19 by the pipette 21. The amount of the cell solution and amount of culture solution to be added are calculated according to the cell count value. The cell culture solution in the tank 19 of 25 mℓ was agitated by the suction and discharge by the pipette 16.

The pipette 16 is provided for taking in and pouring to the 96 well tray. The pipette 16 has its pipe end attached an exchangeable tip of 250 µℓ. In addition, the pipette 16 comprises an X moving unit, Z moving unit for moving the pipette in the vertical direction, a locking unit for locking eight pipette tubes in position and eight cylidine pumps.

The X moving unit is common to the X moving unit of the pipette 6. The X moving unit is supported by six holes and the respective pipets are suspended. Moreover, the X moving unit comprises a ball screw and two linear slide shafts, which are commonly used for the pipette 6. Z moving unit is composed of a ball screw and a linear slide shaft for moving the pipet 16 in the vertical direction. The X and Z moving units are respectively driven by a pulse motor. The locking unit locks the eight pipet tubes by the movement of an air cylinder.

The respective tips mounted to the pipet tubes are changed automatically by the tip exchanger 17. In order to change the eight tips as a unit, eight tips are accommodated in one tip adaptor. The tips are inserted in the holes defined in the adaptor one by one.

The tip exchanger 17 is arranged in the same manner as the tip exchanger 7, therefore, details thereof are omitted.

A tip stocker 24 is provided for storing the new tips. 36 tip adaptors each having eight tips can be installed in one magazine. The tip stocker 24 can accommodate nine magazines.

The tip stocker 24 comprises a magazine transfer unit for moving a shelf for the magazine in the vertical direction, a tip adaptor pushing unit for moving the tip adaptor to the tip taking position where the tip adaptor moving unit can take the tip adaptor, a magazine discharge unit for discharging the empty magazine to the outside of the passage device and a magazine lift unit for lifting the magazine to be discharged.

The magazine transfer unit is formed by a pair of chains for supporting the magazine therebetween horizontally. Said pair of chains are provided with a plurality of brackets for detachably supporting the magazine. The lowest bracket is situated at a level from which the adaptor can be taken out by the tip adaptor pushing unit.

The tip adaptor pushing unit comprises a block member having two poles, a ball screw and two slide shafts. When the ball screw is rotated by a pulse motor the block member is moved to an outlet so that the adaptor can be taken from the magazine. When all of the adaptors are removed from the magazine, the magazine is discharged to the outside of the device, and in turn the next magazine can be transferred to the outlet. In order to discharge the magazine, the magazine lifting unit lifts up the magazine from the bracket and the magazine discharge unit pushes the lifted magazine to the outlet.

The call culture solution in the tank 19 can be agitated by the pipette 16 which is provided with new tips.

In the example, twenty times of agitation were performed using eight tips of 150 µℓ.

After the cell culture solution in the tank 19 is agitated 400 µℓ cell culture solution was taken out by operating eight tips each having 50 µℓ capacity. The 400 µℓ cell culture solution thus taken out was transferred to the tank 18. New culture solution of 24600 µℓ was added to the tank 18 by the pipette 20.

The agitated cell culture solution was taken to the pipet 16 in a rate of 150 µℓ per 1 pipette and the cell culture solutions in the respective pipets were poured to the corresponding wells of the 96 well tray, which is transferred to the tray transferring unit 5 with the respective lids covered on the tray.

In the example, the 96 well tray containing cell culture solution performed by the limiting dilution was cultured for two days. As the result of the observation of the cultured solution as mentioned above, there were 36 wells in which only one colony was present in one well, there were three wells in which two colonies were present in one cell and there were 56 wells in which colony was absent. As mentioned above, according to the passage device of the present invention, a desired effect of the limiting dilution can be obtained. Since whole passage device is covered by the screen bench 30, the system was not contaminated by other microorganisms.

It is possible to subculture by pouring the cells after dilution. In this case, the condition of the cell propagation is monitored by the picture on the television device and the number of cell is counted. Then it is judged whether or not the subculture is necessary, and if subculture is necessary, the subculture is performed, whereby the death of cells due to the full growth can be prevented.

## Claims

1. A cell passage device comprising:
a pipette manipulator having its end mounted with a pipet (6, 16) and movable in three dimensions;
means for sucking and discharging solution to a culture solution vessel;
a first cell solution vessel for retaining cell solution;
a second cell solution vessel for receiving the cell solution of the first vessel through said pipet manipulator; and
means for adding further culture solution in said second vessel by said pipette manipulator,
characterized by means for controlling the pipet and the sucking and discharging means, so as to agitate cell solution in the first or second cell solution vessel.

2. The cell passage device according to claim 1, characterized by means for controlling the pipette manipulator so that said agitation is performed at a plurality of positions in one vessel.

3. The cell passage device according to claim 1, characterized by counting means (2, 3, 34) for counting the number of cells in said first vessel, so that after the cell solution is poured in the second vessel, further amount of the cell culture solution obtained by the result of the counting value is added to the first vessel.

## Patentansprüche

1. Zellendurchlaufgerät, umfassend:
einen Pipettenmanipulator, an dessen Ende eine in drei Richtungen bewegliche Pipette (6, 16) befestigt ist;
Apparaten zum Absaugen und Entleeren einer Lösung in ein Kulturlösungsgefäß;
ein erstes Kulturlösungsgefäß zur Aufnahme der Zellösung des ersten Gefäßes durch den Pipettenmanipulator; und
Apparaten zum Eingeben zusätzlicher Zellösung in das zweite Gefäß durch den Pipettenmanipulator,
**gekennzeichnet** durch
Apparaten zum Steuern der Pipette und der Ansaug- und Entleerungseinrichtungen, derart, daß die Zellösung im ersten oder zweiten Zellösungsgefäß verwirbelt wird.

2. Zellendurchlaufgerät nach Anspruch 1, gekennzeichnet durch eine Apparat zum Steuern des Pipettenmanipulators, derart, daß das Verwirbeln an mehreren Stellen im Gefäß durchgeführt wird.

3. Zellendurchlaufgerät nach Anspruch 1, gekennzeichnet durch Zähleinrichtungen (2, 3, 34) zum Zählen der Anzahl von Zellen im ersten Gefäß, so daß nach dem Einführen der Zellösung in das zweite Gefäß eine weitere Menge der Zellkulturlösung aufgrund des Ergebnisses des Zählwertes zusätzlich in das erste Gefäß eingegeben wird.

## Revendications

1. Appareil à passage de cellules, comprenant :
un manipulateur à pipette dont l'extrémité porte une pipette (6, 16) et qui est mobile suivant trois directions,
un dispositif d'aspiration et d'évacuation d'une solution dans un récipient de solution de culture,
un premier récipient de solution de cellules destiné à contenir une solution de cellules,
un second récipient de solution de cellules destiné à recevoir la solution de cellules du premier récipient par l'intermédiaire du manipulateur à pipette, et
un dispositif destiné à ajouter une solution supplémentaire de culture dans le second récipient à l'aide du manipulateur à pipette,
caractérisé par un dispositif de commande de la pipette et du dispositif d'aspiration et d'évacuation de manière que la solution de cellules soit agitée dans le premier ou le second récipient de solution de cellules.

2. Appareil à passage de cellules selon la revendication 1, caractérisé par un dispositif de commande du manipulateur à pipette de manière que l'agitation soit réalisée en plusieurs positions dans un même récipient.

3. Appareil à passage de cellules selon la revendication 1, caractérisé par un dispositif de comptage (2, 3, 34) destiné à compter le nombre de cellules dans le premier récipient, si bien que, après que la solution de cellules a été déversée dans le second récipient, une quantité supplémentaire de solution de culture de cellules déterminée en fonction du résultat du comptage est ajoutée au premier récipient.
